# EUROPEAN PATENT APPLICATION

(11) **EP 3 375 415 A1**
(43) Date of publication of application: **19.09.2018**
(21) Application number: 17161604.8
(22) Date of filing: 17.03.2017
(51) Int. Cl.: A61F 5/01, A47C 9/02, A61H 3/00, B25J 9/00

(54) **WEARABLE SITTING POSTURE ASSISTING DEVICE**

(71) Applicant: noonee AG, 8630 Rüti ZH (CH)
(72) Inventor: Gunura, Keith, 8003 Zürich (CH); Motovilova, Olga, 8045 Zürich (CH); Vafi, Daniel, 8032 Zürich (CH)
(74) Representative: Daub, Thomas

(57) **Abstract**

The invention relates to a wearable sitting posture assisting device, comprising at least one body connection unit (10a, 12a; 10b), the body connection unit (10a, 12a; 10b) featuring at least one support element (14a; 14b) and at least one body part connector (16a; 16b) for connecting to at least one body part which differs from a foot of a person (200a)

It is proposed that the body part connector (16a; 16b) is connectable to and/or disconnectable from the support element (14a; 14b) in at least one body part connector wearing state.

## Description

### State of the Art

The invention relates to a wearable sitting posture assisting device according to the preamble of claim 1 and relates to a functional module according to claim 14.

A posture assisting device is known from the document WO 2015/028373 A1.

The objective of the invention is, in particular, to provide a generic wearable sitting posture assisting device with improved characteristics regarding comfort. Furthermore, an objective of the invention is, in particular, to provide a wearable sitting posture assisting device which is easy to put on and/or take off. In addition, an objective of the invention is, in particular, to achieve a short time required for putting on and/or taking off a wearable sitting posture assisting device. The objective is achieved according to the invention by the features of claim 1, while advantageous embodiments and further developments of the invention may be gathered from the dependent claims and the further independent claim.

### Advantages of the Invention

The invention relates to a wearable sitting posture assisting device, comprising at least one body connection unit, the body connection unit featuring at least one support element and at least one body part connector for connecting to at least one body part which differs from a foot of a person, in particular to a shank and/or a thigh of a person.

It is proposed that the body part connector is connectable to and/or disconnectable from, in particular entirely disconnectable from, the support element in at least one body part connector wearing state.

By means of the invention, a high degree of comfort, in particular a high degree of handling comfort, can be achieved. Furthermore, a wearable sitting posture assisting device can be provided, which can be put on and/or taken off comfortably and/or easily and/or quickly. Advantageously, a high degree of time-efficiency, in particular if frequently putting on and/or taking off a wearable sitting posture assisting device, can be achieved. Furthermore, a body part connector can be put on and/or taken off unobstructedly by heavy and/or bulky components of a wearable sitting posture assisting device. In particular, an intuitive handlability can be achieved. Furthermore, a body part connector can be adjusted and/or fastened in a comfortable and/or unobstructed manner.

A "wearable sitting posture assisting device" is herein to be understood as a device which is configured for receiving a weight force of a person in a sitting posture or in a partly sitting posture and in particular for transmitting the weight force to a ground. In particular, an angle between a thigh and a shank of at least one leg of the person in the sitting posture is no greater than 130°, preferably no greater than 120° and advantageously no greater than 110° and/or no smaller than 60°, preferably no smaller than 70° and advantageously no smaller than 80°. In particular, the angle between the thigh and the shank of the person is approximately 90° in the sitting posture. In particular, an angle between a thigh and a shank of the person in the partly sitting posture is no greater than 170°, preferably no greater than 160° and advantageously no greater than 150° and/or no smaller than 100°, preferably no smaller than 110° and advantageously no smaller than 120°. In particular, the angle between the thigh and the shank of the person is approximately 130° in the partly sitting posture. It is conceivable that the partly sitting posture is a posture in which the person leans forward while partly bending his knees. In particular, a person wearing the wearable sitting posture assisting device is enabled to sit and/or to partly sit and/or to sit down on the wearable sitting posture assisting device, wherein at least a portion of the weight force is counteracted by the wearable sitting posture assisting device, and/or wherein the person counteracts only a fraction of the weight force using his muscles. In particular, the wearable sitting posture assisting device is configured for being worn by the person while the person is standing and/or while the person is walking. Advantageously, the wearable sitting posture assisting device is configured for supporting different sitting postures and/or partly sitting postures, which are in particular characterized by different sitting angles.

The wearable sitting posture assisting device in particular defines a sitting direction. Preferably, the person faces and/or looks in the sitting direction when sitting or partly sitting on the wearable sitting posture assisting device and facing forward. In particular, the sitting direction is oriented parallel to a floor on which the person is standing and/or sitting and/or walking when wearing the wearable sitting posture assisting device. In particular, the wearable sitting posture assisting device defines a walking direction. Preferably, the person faces in the walking direction when walking and/or standing with the wearable sitting posture assisting device and facing forward. In particular, the walking direction is oriented parallel to a floor on which the person is standing and/or sitting and/or walking when wearing the wearable sitting posture assisting device. In particular, the wearable sitting posture assisting device is only designed to receive and transmit the weight force. Preferably, the wearable sitting posture assisting device is not designed to generate a controllable force which is configured to assist a person while walking, standing or lifting some loads. In this context, "configured" is in particular to mean specifically programmed, designed and/or equipped. By an object being configured for a certain function is in particular to be understood that the object implements and/or fulfills said certain function in at least one application state and/or operating state.

Preferably, the wearable sitting posture assisting device comprises at least one leg unit. In particular, the wearable sitting posture assisting device comprises at least one additional leg unit. In particular, the leg unit comprises at least one, preferably one, upper leg and/or at least one, preferably one, lower leg and/or may comprise at least one, preferably one, foot unit, and/or comprises at least one, preferably one, ground contact unit. Advantageously, the upper leg is connected to the lower leg, in particular via at least one, in particular one, knee joint. Preferably, the foot unit is connected to the lower leg. Advantageously, the ground contact unit is connected to the lower leg and/or to the foot unit. It is conceivable that the ground contact unit is at least partly implemented integrally with the lower leg and/or at least partly implemented integrally with the foot unit. It is also conceivable that the foot unit is at least partly implemented integrally with the lower leg. Preferably, the wearable sitting posture assisting device comprises at least one upper body wearing unit. In particular, the leg unit is connected to the upper body wearing unit, preferably via at least one connection strap. In this context, the term "a first object and a second object being at least partly implemented integrally" is in particular to mean that at least one component of the first object and at least one component of the second object are implemented integrally with each other. "Implemented integrally" is in particular to mean, in this context, connected at least by substance-to-substance bond, e.g., by a welding process, an adhesive bonding, an injection-molding process and/or by another process that is deemed expedient by a person having ordinary skill in the art. Advantageously, "implemented integrally" could in particular mean made of one piece. "Made of one piece" is, in particular, to mean, in this context, manufactured from one single piece, e.g., by production from one single cast and/or by manufacturing in a one-component or multi-component injection-molding process, and advantageously from a single blank.

Preferably, the wearable sitting posture assisting device comprises two leg units. Advantageously, the leg unit and the additional leg unit are implemented identically. It is also conceivable that the leg unit and the additional leg unit are implemented mirror-symmetrically with respect to each other. It is conceivable that the leg unit is configured for being worn on a left leg and the additional leg unit is configured for being worn at a right leg, or vice versa. Advantageously, the leg unit is configured for being worn either on a left leg or on a right leg. Further advantageously, the additional leg unit is configured for being worn on a left leg or on a right leg. Preferably, the additional leg unit is connected to the upper body wearing unit, preferably via at least one connection strap. In particular, the person wearing the wearable sitting posture assisting device wears the leg unit, in particular solely, on a first leg, for instance a left leg or a right leg. In particular, the person wearing the wearable sitting posture assisting device wears the additional leg unit, in particular solely, on a second leg, for instance a right leg or a left leg. Advantageously, the leg unit is arranged on a rear side of the leg on which the leg unit is worn. Further advantageously, the additional leg unit is arranged on a rear side of the leg on which the additional leg unit is worn. In particular, the leg units of the wearable sitting posture assisting device are arranged on respective rear sides of the legs of the person when the person is sitting and/or partly sitting on the wearable sitting posture assisting device and/or standing and/or walking with the wearable sitting posture assisting device. Preferably, the person wearing the wearable sitting posture assisting device wears the upper body wearing unit on his upper body. Advantageously, the upper body wearing unit is implemented as a belt and/or as braces and/or as suspenders. The wearable sitting posture assisting device enables the person wearing it to walk around, to stand, to sit down on the wearable sitting posture assisting device if required or desired and to stand up after sitting or partly sitting on the wearable sitting posture assisting device.

Preferably, the upper leg comprises at least one thigh connection unit for connecting to a thigh of the person. Preferably, the thigh connection unit features at least one thigh strap. In particular, the upper leg comprises a seat unit configured for providing at least one sitting surface for the person, in particular in case the person is sitting or partly sitting on the wearable sitting posture assisting device, preferably for the thigh and/or at least a lower portion of a buttock of the person, wherein "buttock" is preferably to mean one cheek of the buttocks. Preferably, the seat unit comprises at least one sitting element which features the sitting surface. Advantageously, the seat unit is in contact with the thigh of the person in case the person is sitting or partly sitting on the wearable sitting posture assisting device. Preferably, the seat unit is arranged on a rear side of the thigh of the person in case the person is standing or walking with the wearable sitting posture assisting device.

Advantageously, the upper leg comprises at least one upper leg support. Preferably, the seat unit is connected to the upper leg support. Advantageously, the thigh connection unit and/or the thigh strap is connected to the upper leg support. In particular, the upper leg support is implemented as a frame element. Preferably, the upper leg support is implemented as an elongate element. Advantageously, the upper leg features at least one upper leg longitudinal axis which is oriented at least substantially parallel to a longitudinal axis of the thigh of the person. Preferably, a main extension direction of the upper leg support is oriented at least substantially parallel, or parallel, to the upper leg longitudinal axis. In particular, the upper leg support is at least partly, preferably at least to a large extent, advantageously completely made of plastic. It is also conceivable that the upper leg support is at least partly, preferably at least to a large extent, advantageously completely made of metal, in particular made of a light metal or a light alloy, for instance aluminum and/or titanium and/or beryllium and/or scandium or other suitable metals. It is further conceivable that the upper leg support is at least partly, preferably at least to a large extent, advantageously completely made of a composite material, in particular a fiber reinforced composite material and/or a fiber reinforced plastic and/or a carbon fiber reinforced material and/or a carbon fiber reinforced polymer and/or a fiber reinforced thermoplastic. The term "at least to a large extent" is in particular to mean to an extent of at least 55 %, preferably to an extent of at least 65 %, further preferably to an extent of at least 75 %, advantageously to an extent of at least 85 % and further advantageously to an extent of at least 95 %. In this context "at least substantially parallel" is in particular to be understood as an orientation of a direction with respect to a reference direction, in particular in a plane, wherein the direction has a deviation from the reference direction in particular of less than 15º, advantageously of less than 10º and particularly advantageously of less than 2º. A "main extension direction" of an object is, in particular, to be understood, in this context, as a direction extending in parallel to a largest side of an imaginary rectangular cuboid which only just entirely encloses the object.

Preferably, the lower leg is arranged on a rear side of the shank of the person in at least one wearing state. In particular, the lower leg comprises at least one lower leg support. Advantageously, the lower leg support is implemented as a frame element. Preferably, the lower leg support is implemented as an elongate element. In particular, the lower leg features at least one lower leg longitudinal axis which is oriented at least substantially parallel to a longitudinal axis of the shank of the person. Preferably, a main extension direction of the lower leg support is oriented at least substantially parallel, or parallel, to the lower leg longitudinal axis. Advantageously, the lower leg longitudinal axis is oriented at least substantially parallel, or parallel, to the upper leg longitudinal axis.

Preferably, the upper leg and the lower leg together define a sitting angle. Advantageously, the sitting angle is an angle included by the upper leg longitudinal axis and the lower leg longitudinal axis, in particular on a rear side of the upper leg and the lower leg. In particular, in the sitting posture the sitting angle is no greater than 130°, preferably no greater than 120° and advantageously no greater than 110° and/or no smaller than 60°, preferably no smaller than 70° and advantageously no smaller than 80°. In particular, the sitting angle is approximately 90° in the sitting posture. In particular, in the partly sitting posture the sitting angle is no greater than 170°, preferably no greater than 160° and advantageously no greater than 150° and/or no smaller than 100°, preferably no smaller than 110° and advantageously no smaller than 120°. In particular, the sitting angle is approximately 130° in the partly sitting posture. Preferably, the sitting angle equals the angle between the thigh and the shank of the person. In particular, the sitting angle is approximately 180° in a standing posture.

In particular, the knee joint pivotably connects the lower leg to the upper leg, preferably pivotably about a knee joint axis. Advantageously, the knee joint axis is oriented at least substantially perpendicularly, or perpendicularly, to the upper leg longitudinal axis and/or to the lower leg longitudinal axis. Preferably, the knee joint axis is oriented at least substantially perpendicularly, or perpendicularly, to the sitting direction. Advantageously, the upper leg support and the lower leg support together implement at least a portion of the knee joint, or the knee joint. Preferably, a value of the sitting angle corresponds to a value of a knee joint position of the knee joint. In this context "at least substantially perpendicularly" is in particular to mean an orientation of a direction with respect to a reference direction, in particular in a plane, wherein the direction and the reference direction include an angle which deviates from an angle of 90° by no more than 15º, advantageously by no more than 10º and particularly advantageously by no more than 2º.

Preferably, the wearable sitting posture assisting device comprises at least one locking unit, which is configured for locking the upper leg with respect to the lower leg and/or the knee joint in a certain sitting angle and/or is configured for defining a smallest sitting angle. Advantageously, the locking unit is configured for locking the knee joint in different sitting angles, and/or for defining different smallest sitting angles, which sitting angles can be preferably chosen by the person. It is conceivable that the locking unit is configured for allowing to increase the sitting angle in a locked state. In particular, the person is enabled to stand up when the locking unit is in the locked state. Preferably, the locking unit is configured for enabling the person to sit down again at the defined smallest sitting angle after standing up with the locking unit still defining the same smallest sitting angle. Advantageously, the locking unit comprises at least one blocking element which is configured for blocking and/or unblocking the knee joint and/or for fixating the sitting angle and/or for defining a smallest sitting angle. Preferably, the blocking element is implemented as a spring, in particular as a gas spring. Advantageously, the blocking element is connected to the upper leg, in particular to the upper leg support, and to the lower leg, in particular to the lower leg support. Preferably, the blocking element is configured for damping a movement of the upper leg with respect to the lower leg during sitting down and/or during standing up. Advantageously, the locking unit features at least one actuation element, which is configured for actuating the blocking element. Preferably, the actuation element is configured for enabling the person to block or unblock the blocking element. Advantageously, the actuation element is a mechanical actuation element. It is also conceivable that the actuation element is an electronic actuation element. It is further conceivable that the locking unit comprises at least one control unit, which is configured for detecting a sitting-down condition when the person sits down and/or detecting a standing-up condition when the person stands up and/or triggering the actuation element according to a requirement for blocking the blocking element.

In particular, the wearable sitting posture assisting device may comprise a foot unit, which foot unit may be configured for connecting to a foot and/or shoe of the person and/or which foot unit may be connected to a foot and/or shoe of the person. Preferably, the foot unit may comprise at least one foot connector for connecting to the foot and/or shoe of the person. Advantageously, the foot connector may feature at least one shoe strap. In particular, the foot connector may feature at least one upper strap, which advantageously runs across an instep of the foot and/or shoe which the foot unit is connected to. Preferably, a foot connector may feature at least one lower strap, which advantageously runs across a sole of the foot and/or shoe which the foot unit connected to. Preferably, a foot connector may be configured for being worn on a foot and/or shoe. Advantageously, the foot unit may comprise at least one foot unit support. Preferably, the foot connector may be connected to the foot unit support. Advantageously, the foot unit support could comprise at least one bracket and/or the foot unit support element could be implemented as a bracket. Preferably, the shoe strap, in particular the upper strap and/or the lower strap, may be connected to the bracket. It is conceivable that the foot unit support element could be at least partly implemented integrally, or implemented integrally, with the lower leg, in particular with the lower leg support.

Additionally or alternatively, in particular alternatively to the foot unit, the wearable sitting posture assisting device, in particular the leg unit, comprises at least one shank connection unit for connecting to the shank of the person. In particular, the wearable sitting posture assisting device may comprise the shank connection unit and be free of a foot unit and/or a foot connector.

In particular, the ground contact unit comprises at least one ground contact element. Preferably, the ground contact element features at least one ground contact surface, which is advantageously configured for contacting a ground when the person is sitting or partly sitting on the wearable sitting posture assisting device. Advantageously, the ground contact surface is bent and/or curved, in particular convexly bent and/or convexly curved. However, it is also conceivable that at least a portion of or the entire ground contact surface is at least substantially flat or flat. In particular, the ground contact element is at least partly, preferably at least to a large extent, advantageously completely made of rubber. Preferably, a weight force of the person is transmitted from the seat unit to the upper leg support and/or from the upper leg support to the knee joint and/or from the knee joint to the lower leg support and/or from the lower leg support to the ground contact element and/or from the ground contact element to the ground. In particular, the weight of the person is additionally transmitted to the ground via the foot and/or shoe of the person. Preferably, the ground contact element is arranged on a rear side of the foot and/or shoe of the person. When the person is sitting or partly sitting on the wearable sitting posture assisting device, the foot and/or shoe of the person is in contact with the ground in addition to the ground contact element. Preferably, the ground contact element is arranged contactlessly with respect to the ground when the person is walking or standing while wearing the sitting posture assisting device.

In particular, the body part is not a foot. Advantageously, the body connection unit is configured for at least partly connecting, in the wearing state, at least a portion of the wearable sitting posture assisting device, in particular the leg unit or the additional leg unit, to the person. A high degree of handling comfort can be achieved and/or a leg unit which is easy to put on and/or take off can be provided if the support element is connected to the leg unit. In particular, the support element is mounted and/or fixated to the leg unit. It is conceivable that the support element is at least partly implemented integrally with the leg unit, in particular with the upper leg support, the lower leg support, the foot unit support and/or the seat unit.

Preferably, a position of the body part connector with respect to the thigh and/or shank of the person which the body part connector is connected to is at least substantially fixed, or fixed, in the wearing state. In particular, the body part connector is configured for connecting to the thigh and/or the shank of the person such that a position of the body part connector with respect to the thigh and/or shank of the person to which the body part connector is connected is changed by no more than 10 cm, preferably by no more than 5 cm and further preferably by no more than 3 cm while the person is wearing the wearable sitting posture assisting device. Preferably, the body part connector comprises at least one body connection element for connecting to the thigh and/or shank of the person, in particular to a piece of clothing. In particular, the body connection element is configured for at least partly encompassing the body part of the person in the body part connector wearing state. The body connection element may for instance comprise at least one strap element, at least one bracket element, at least one clamp element, at least one fastening element and/or the like. It is conceivable that the body part connector is implemented integrally. It is also conceivable that the body part connector is implemented modularly, in particular with the body connection element being implemented as an exchangeable modular portion of the body part connector, which advantageously allows an adjustment to at least one body dimension of the person wearing the wearable sitting posture assisting device. Preferably, the body part connector is configured for connecting either to a shank or to a thigh of the person. It is also conceivable that the body part connector is configured for connecting to a thigh and a shank of the person, in particular to a knee and/or a knee region of the person. It is further conceivable that the body part connector is implemented as an upper body wearing element and/or is part of the upper body wearing unit. In particular, the body part connector may be implemented as a belt and/or a vest. In particular in this case, the body connection unit may implement at least a portion of or may be implemented as the upper body wearing unit.

Preferably, the body part connector is fixed to the support element in a connected state of the body part connector. In particular, a position of the body part connector with respect to the support element is fixed, at least within a distance of no more than 5 cm, preferably of no more than 3 cm and further preferably of no more than 2 cm and/or at least in one direction, preferably at least in at least two directions which are oriented perpendicularly to each other. In particular, in the connected state a connection between the body part connector and the support element is configured for withstandding a force, in particular a pull force exerted onto the body part connector, in particular in a direction that is perpendicular to a main extension plane of the connection, of at least 50 N, preferably of at least 100 N, further preferably of at least 200 N, advantageously of at least 300 N and further advantageously of at least 400 N. It is conceivable that a maximum connection force of the connection of the body part connector and the support element is adjustable, for instance allowing a connection force to be adjusted to a weight and/or a strength of the person and/or to an area of application of the wearable sitting posture assisting device. Advantageously, the body part connector is entirely disconnected from the support element in a disconnected state of the body part connector. It is conceivable that the body connection unit, in particular the support element and/or the body part connector, comprises at least one actuation element for locking and/or unlocking the connection between the body part connector and the support element. In particular, connecting the body part connector to the support element may encompass bringing the body part connector in contact with the support element and/or fastening the body part connector to the support element and/or establishing a locking. It is also conceivable that the body part connector and/or the support element implements or the body part connector and the support element together implement at least one self-locking and/or self-unlocking connection. In particular, the connection between the body part connector and the support element may be configured for self-unlocking and/or for self-releasing in case a threshold force exerted onto the body part connector is exceeded and/or in an emergency. It is further conceivable that the threshold force is adjustable. Furthermore, it is conceivable that the body connection unit comprises at least one sensor unit for detecting at least one parameter indicating an emergency. In particular in this case, the body connection unit may comprise at least one automatic actuation unit for releasing the connection of the body part connector to the support element in an, in particular detected, emergency state. As a result, a high degree of safety can be achieved and/or injuries can be avoided. A "main extension plane" of an object is, in particular, to be understood as a plane extending parallel to a largest side of an imaginary rectangular cuboid which only just entirely encloses the object and preferably extends through a geometric center of the object.

In particular, the body part connector is connected to the body part of the person in the body part connector wearing state. In the body part connector wearing state the body part connector may be connected to or disconnected from the support element. Preferably, in at least one normal wearing state of the wearable sitting posture assisting device, a force and/or a movement of the body part is at least partly transmitted from the body part connector to the support element and/or from the support element to the leg unit.

In a further embodiment of the invention it is proposed that the body part connector and the support element together implement at least one quick connector. For instance, the quick connector may be implemented as a mechanical quick connector. It is conceivable that the quick connector is implemented as a click-in connector, a snap connector, a bayonet-type connector, a plug-in connector, a socket fastener, a key lock fastener, a hook-and-loop fastener, a touch fastener, or the like. The quick connector may comprise at least one actuation element, in particular a mechanical actuation element, for opening and/or closing the connection between the body part connector and the support element. Preferably, the actuation element is configured for being actuated with one hand and/or finger, for instance with a thumb. In particular, the body part connector comprises at least one first quick-connection element. Furthermore, the support element in particular comprises at least one second quick-connection element. Preferably, the first quick-connection element and the second quick-connection element together implement the quick connector. As a result, a connection can be established and/or released fast and reliably. In particular, a high degree of time-efficiency can be achieved.

In another embodiment of the invention it is proposed that the quick connector is self-fastening and/or self-connecting. Preferably, the quick connector is self-centering. For instance, the quick connector may comprise at least one catching element and/or at least one centering element for catching and/or centering at least a portion of the body part connector and/or the support element in at least one proximity state of the body part connector with respect to the support element. For instance, the proximity state may be a transition state prior to connecting the quick connector. In particular, in the proximity state a distance between the body part connector and the support element is no greater than 15 cm, preferably no greater than 10 cm and further preferably no greater than 5 cm and/or no less than 1 cm, preferably no less than 2 cm and further preferably no less than 3 cm. Advantageously, the first quick-connection element and/or the second quick-connection element is configured for catching and/or centering the second quick-connection element and/or the first quick-connection element, respectively. Preferably, the quick connector is configured for opening and/or closing the connection between the body part connector and the support element without touching the support element and/or the body part connector with one or more hands. In particular, the quick connector is configured for automatically closing the connection between the body part connector and the support element, in particular in case the body part connector is in the proximity state. As a result, a wearable sitting posture assisting device can be easily put on and/or taken off. In particular, a person can simply approach the wearable sitting posture assisting device and is automatically connected to it, advantageously without a necessity for manually actuating connection elements with his hands.

Operating errors and in particular resulting potential accidents can be avoided, in particular, if the quick connector is configured for providing at least one inadvertent-error-prevention constraint. Preferably, the quick connector is of a poka yoke type. Advantageously, the quick-connection elements together implement a poka yoke connection. For instance, the quick connector is implemented in such a way that it prevents erroneous connection of the body part connector to the support element, for instance an upside-down connection, a reverse connection or a connection of the body part connector to a wrong support element. For instance, it is conceivable that the body connection unit is implemented as a left-leg body connection unit or as a right-leg body connection unit. In particular, it is conceivable that the quick connector prevents connection of a left-side body part connector to a right-side support element or vice versa.

A fast and/or reliable connector for a wearable sitting posture assisting device can be provided, in particular, if the body part connector and the support element together implement at least one magnetic connection. Preferably, the quick connector is implemented as a magnetic quick connector. In particular, the quick connector implements the magnetic connection. Advantageously, the first quick-connection element and/or the second quick-connection element comprises at least one magnet and/or at least one magnetizable, in particular ferromagnetic, portion. It is conceivable that the magnetic connection comprises a plurality of magnets, which are preferably arranged on at least one connection surface. Preferably, the connection surface is configured for connecting to at least one second connection surface, which may comprise a plurality of magnets and/or magnetizable elements, in particular ferromagnetic elements, which are in a connected state preferably respectively assigned to the magnets of the connection surface. In particular, a connection analogous to a hook-and-loop fastener or a touch fastener can be implemented using magnets, which is in particular strong against a pull in a first direction and/or which can be easily released by pull in a second direction, in particular sideways, and/or by peeling one connection surface off the other one.

In another embodiment of the invention it is proposed that the body part connector and the support element, in particular the first quick-connection element and the second quick-connection element, together implement at least one form-fit connection. Preferably, the form-fit connection comprises at least one protrusion element and/or at least one recess element featuring at least one recess. Advantageously, the protrusion element is at least partly insertable into the recess of the recess element, further advantageously in a direction that is perpendicular to the sitting direction. Preferably, in a connected state of the body part connector the form-fit connection is configured for at least partly transmitting a pull force exerted onto the body part connector from the protrusion element, in particular from at least one lateral surface of the protrusion element, to the recess element, in particular to at least one lateral surface of the recess element which delimits the recess. In particular, the quick connector implements the form-fit connection. Preferably, the form-fit connection provides the inadvertent-error-prevention constraint. As a result, a reliable connection, which is in particular protected against inadvertent shifting or any inadvertent movement, can be provided. Furthermore, accidental disconnection of a body part connector from a support element can be advantageously avoided.

A high degree of handling comfort regarding putting a leg unit on a leg can be achieved, in particular, if the body part connector is implemented as a thigh connector and/or as a shank connector. In particular, the support element is connected to the upper leg in case the body part connector is implemented as a thigh connector. Furthermore, the support element is in particular connected to the lower leg in case the body part connector is implemented as a shank connector.

A high degree of wearing comfort can be achieved and/or a person wearing a wearable sitting posture assisting device can be enabled to at least slightly move his legs and/or feet while sitting on the wearable sitting posture assisting device, in particular, if the support element is at least partly macroscopically deformable, in particular deformable repeatedly and/or without damage. Furthermore, components of a connector in particular self-adjust while connecting. In particular, the support element is at least partly flexible and/or elastic. Advantageously, the support element is at least partly stretchable and/or compressible and/or twistable. Preferably, the support element comprises at least one compensation element, which is, in particular repeatedly and/or without damage, macroscopically deformable. For instance, the compensation element may be implemented as a strap element and/or a fixing element. Additionally or alternatively, it is conceivable that the compensation element is implemented as a spring element and/or a damping element and/or a joint element, in particular a ball-joint element. Preferably, the compensation element is at least partly, preferably at least to a large extent, made of rubber. In this context, a "macroscopically deformable object" is, in particular, an object having at least one extension in at least one direction, which extension can be, in particular temporarily and/or without damage, altered by at least 1 %, preferably by at least 5 %, further preferably by at least 20 % and advantageously by at least 50 % when a force is applied to the object that is no greater than 1000 N mm⁻², preferably no greater than 100 N mm⁻² and advantageously no greater than 10 N mm⁻². Advantageously, the compensation element connects, in particular directly connects, the second quick-connection element to the leg unit.

It is further proposed that the wearable sitting posture assisting device comprises at least one additional body connection unit, the additional body connection unit featuring at least one additional support element and at least one additional body part connector for connecting to at least one additional body part of the person, wherein the additional body part connector is connectable to and/or disconnectable from, in particular entirely disconnectable from, the additional support element. Advantageously, the additional body connection unit is at least partly implemented analogously to the body connection unit. In particular, individual features, in particular an arbitrary combination of individual features, or all features described for the body connection unit are transferable to the case of the additional body connection unit. As a result, a high degree of comfort and/or time efficiency can be achieved.

Furthermore, it is proposed that the body connection unit and the additional body connection unit are configured for connecting to different kinds of body parts. In particular, the body connection unit is configured for connecting to the thigh of the person and the additional body connection unit is configured for connecting to the shank or the foot of the person, or vice versa. It is also conceivable that the body connection unit or the additional body connection unit are configured for connecting to an upper body. Preferably, the wearable sitting posture assisting device, in particular the leg unit, comprises precisely one body connection unit for each body part to which the wearable sitting posture assisting device, in particular the leg unit, is connected, each of these body connection units in particular comprising at least one body part connector and at least one support element, wherein said body part connector is connectable to and/or disconnectable from said support element. Advantageously, the thigh connection unit and/or the shank connection unit are respectively implemented as a body connection unit according to the invention. Alternatively or additionally, the foot connector and/or the upper body wearing unit are implemented as a body connection unit according to the invention. Preferably, the leg unit comprises a thigh connection unit and either a foot connector or a shank connector. As a result, an entire leg unit and/or an entire wearable sitting posture assisting device can be put on and/or taken off in a time-efficient and/or comfortable way.

The invention further encompasses a body part connector for the wearable sitting posture assisting device, which in particular comprises the support element. In particular, the body part connector is configured for implementing the body connection unit together with the support element of the wearable sitting posture assisting device. Preferably, the body part connector is configured for implementing a wearable sitting posture assisting device together with a functional module comprising the leg unit and the support element. As a result, a high degree of versatility can be achieved, for instance by providing different body part connectors, which are in particular different regarding a size, a shape, a strength or the like, for alternatively connecting to the same leg unit and/or functional unit of a wearable sitting posture assisting device.

Furthermore, the invention encompasses a functional module, which is in particular configured for the wearable sitting posture assisting device, comprising at least one functional unit, the functional unit being configured for implementing in at least one wearing state at least one posture assisting function, in particular at least one sitting posture assisting function, for a person, and comprising at least one support element, which is configured for connecting to and/or disconnecting from at least one body part connector in at least one body part connector wearing state. Advantageously, the functional module is a component of the wearable sitting posture assisting device. Further advantageously, the functional module is configured for implementing at least a portion of the wearable sitting posture assisting device, preferably together with the body part connector. Preferably, the functional unit comprises the leg unit. Further preferably, the support element is connected to the functional unit, in particular to the leg unit. In particular, the functional unit may comprise the additional leg unit. Further preferably, the functional module is configured for implementing the wearable sitting posture assisting device together with the body part connector. As a result, a fully functional leg unit and/or a functional module of a wearable sitting posture assisting device may be used in combination with different body part connectors. Furthermore, a fully functional leg unit and/or a functional module of a wearable sitting posture assisting device can be provided which is easy to handle and/or quick to put on and/or to take off.

Furthermore, a system is proposed, comprising the functional module and the body part connector. In addition, a system is proposed, comprising the functional module, the body part connector and an alternative body part connector, which is connectable to and/or disconnectable from the support element of the functional unit alternatively to the body part connector.

The invention also comprises a method for putting on and/or taking off the wearable sitting posture assisting device, comprising at least one support element and at least one body part connector, wherein the body part connector is connected to and/or disconnected from the support element in at least one body part connector wearing state. Preferably, for putting on the wearable sitting posture assisting device, the body part connector is put on the body part prior to connecting the body connector to the support element. In particular, the body part connector is connected to the functional module in a body part connector wearing state. Advantageously, for taking off the wearable sitting posture assisting device, the body part connector is disconnected from the support element prior to disconnecting the body part connector from the body part.

Herein, the wearable sitting posture assisting device and the method according to the invention are not to be limited to the application and implementation described above. In particular, for the purpose of fulfilling a functionality herein described, the wearable sitting posture assisting device and the method according to the invention may comprise a number of respective elements, structural components, units and/or steps that differs from the number mentioned herein. Furthermore, regarding the value ranges mentioned in this disclosure, values within the limits mentioned are to be understood to be also disclosed and to be used as applicable.

### Drawing

Further advantages may become apparent from the following description of the drawing. In the drawing exemplary embodiments of the invention are shown. The drawing, the description and the claims contain a plurality of features in combination. The person having ordinary skill in the art will purposefully also consider the features separately and will find further expedient combinations.

If there is more than one specimen of a certain object, in some instance only one of these may be given a reference numeral in the figures and in the description. The description of this specimen may be correspondingly transferred to the other specimens of the object.

It is shown in:
- Fig. 1: a person wearing a wearable sitting posture assisting device, in a schematic lateral view,
- Fig. 2: the person wearing the wearable sitting posture assisting device, in a schematic front view,
- Fig. 3: the person wearing the wearable sitting posture assisting device, in a schematic rear view,
- Fig. 4: the wearable sitting posture assisting device in a disconnected state from a leg of the person, in a schematic lateral view,
- Fig. 5: a portion of a body connection unit of the wearable sitting posture assisting device, in a schematic sectional view, and
- Fig. 6: a leg unit of an alternative wearable sitting posture assisting device, in a schematic lateral view.

### Description of the Exemplary Embodiments

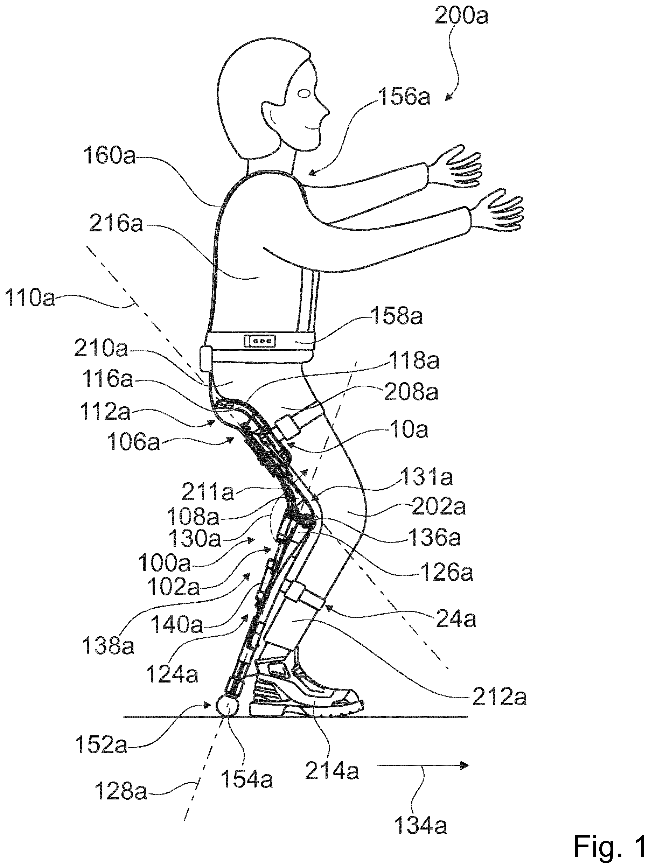

Fig. 1 shows a person 200a wearing a wearable sitting posture assisting device 100a. The wearable sitting posture assisting device 100a is configured for receiving a weight force of the person 200a in a sitting posture or in a partly sitting posture. In fig. 1 the person 200a is shown in a partly sitting posture. In the partly sitting posture a knee 202a of the person 200a is partly bent. In a sitting posture the knee 202a is bent more strongly than in the partly sitting posture. The wearable sitting posture assisting device 100a is configured for allowing the person 200a to sit down on it in different sitting postures and in different partly sitting postures. Furthermore, the wearable sitting posture assisting device 100a is configured for allowing the person 200a to walk while wearing the wearable sitting posture assisting device 100a. In addition, the wearable sitting posture assisting device 100a is configured for allowing the person 200a to stand and/or stand up and/or sit down and/or walk while wearing the wearable sitting posture assisting device 100a.

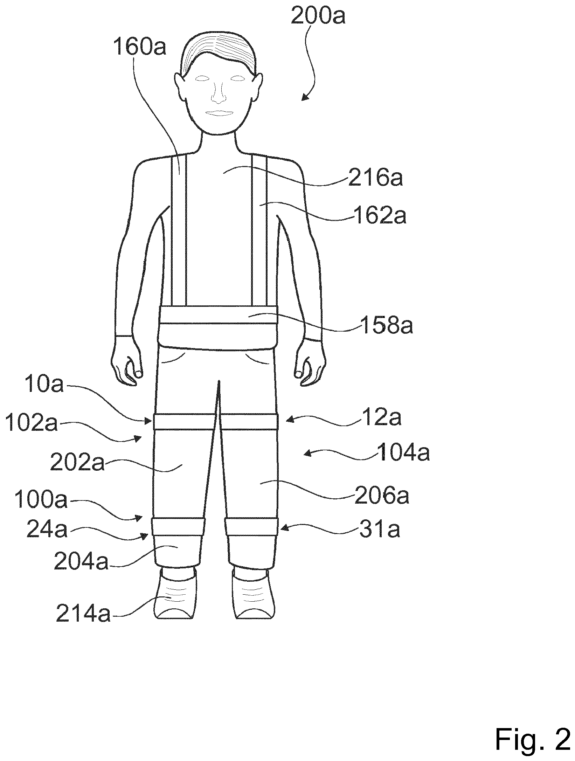

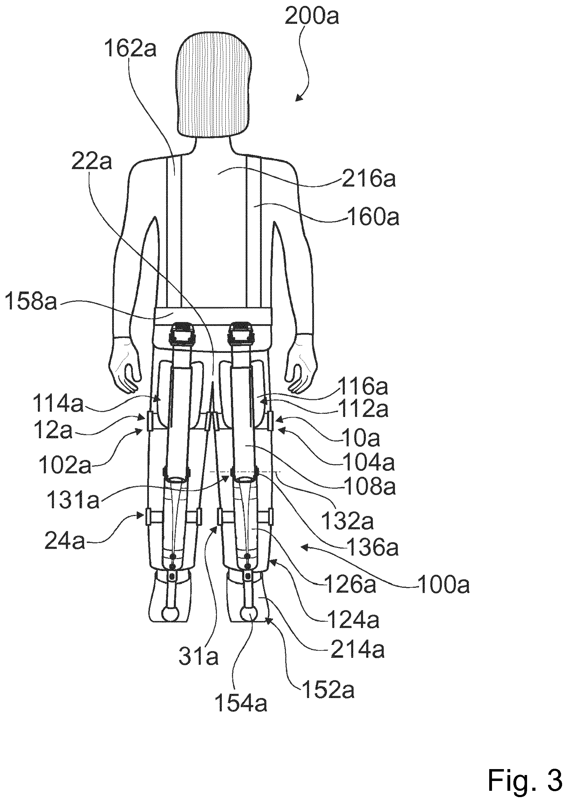

Fig. 2 shows the person 200a wearing the wearable sitting posture assisting device 100a, in a schematic front view. Fig. 3 shows the person 200a wearing the wearable sitting posture assisting device 100a, in a schematic rear view. In figs 1 to 3 the wearable sitting posture assisting device 100a is shown in a normal wearing state. The normal wearing state encompasses a condition in which the person 200a is sitting or partly sitting on the wearable sitting posture assisting device 100a, a condition in which the person 200a is standing up, a condition in which the person 200a is sitting down, a condition in which the person 200a is standing, and a condition in which the person 200a is walking, in each case while wearing the wearable sitting posture assisting device 100a. In the case shown the person 200a is wearing the wearable sitting posture assisting device 100a in a factory building, in particular while working at an assembly line. In a similar fashion it is conceivable that the person 200a wears the wearable sitting posture assisting device 100a in an office building, in a factory building, in a service building, outdoors, at work, at home, while working, during breaks, etc. Advantageously, the person 200a wears the wearable sitting posture assisting device 100a during an activity which requires the person 200a to repeatedly sit down and/or partly sit down and/or stand up and/or stand and/or walk. The person 200a can then sit down on the wearable sitting posture assisting device 100a when required, stand up while wearing the wearable sitting posture assisting device 100a when required, and walk while wearing the wearable sitting posture assisting device 100a when required.

The wearable sitting posture assisting device 100a comprises a leg unit 102a. Furthermore, the wearable sitting posture assisting device 100a comprises an additional leg unit 104a. The additional leg unit 104a is implemented identically and/or analogously to the leg unit 102a. Therefore, in the following only the leg unit 102a is described in detail. The description of the leg unit 102a is to be understood as transferable to the additional leg unit 104a. It is also conceivable that an additional leg unit is implemented mirror-symmetrically to the leg unit. In particular, it is conceivable that a leg unit and an additional leg unit are implemented as a right leg unit and a left leg unit, respectively, or vice versa.

In the case shown, the person 200a wears the leg unit 102a on a right leg 204a. The leg unit 102a is arranged on a rear side 211 a of the leg 204a of the person 200a. Furthermore, the person 200a wears the additional leg unit 104a on a left leg 206a. It is also conceivable that a person wears a leg unit on a left leg and an additional leg unit on a right leg. Furthermore, it is conceivable that a person only wears one leg unit. In addition, it is conceivable that a wearable sitting posture assisting device comprises only one leg unit. It is also conceivable that a leg unit is arranged on a lateral side of a leg and/or on a front side of a leg and/or between two legs of a person.

The person 200a sits or partly sits on the wearable sitting posture assisting device 100a in a sitting direction 134a. The person 200a faces and/or looks in the sitting direction 134a when facing forward. The sitting direction 134a is oriented parallel to a ground on which the person 200a is sitting or walking or standing.

The leg unit 102a comprises an upper leg 106a. The upper leg 106a comprises an upper leg support 108a. The upper leg 106a has an upper leg longitudinal axis 110a. The upper leg longitudinal axis 110a is oriented parallel to a plane defined by the sitting direction 134a and a surface normal of a ground the wearable sitting posture assisting device 100a is standing on in the normal wearing state. The upper leg support 108a has a main extension direction which is oriented parallel to the upper leg longitudinal axis 110a. The upper leg longitudinal axis 110a is oriented parallel to a main extension direction of a thigh 208a of the leg 204a of the person, in particular when the person 200a sits, and/or partly sits and/or walks and/or stands up and/or stands while wearing the wearable sitting posture assisting device 100a.

The upper leg 106a comprises a seat unit 112a. The seat unit 112a is connected to the upper leg support 108a. In the partly sitting posture and/or in the sitting posture the person 200a sits on the seat unit 112a. In the case shown the person 200a sits on the seat unit 112a and on a seat unit 114a of the additional leg unit 104a in the partly sitting posture. The seat unit 112a comprises a sitting element 116a. The sitting element 116a contacts the thigh 208a of the person 200a. Furthermore, in the sitting posture and/or in the partly sitting posture the sitting element 116a contacts a buttock 210a of the person 200a. The seat unit 112a comprises a sitting surface 118a. The sitting element 116a comprises the sitting surface 118a. The sitting surface 118a is configured for allowing the person 200a to sit down on it with his thigh 208a and/or with his buttock 210a. A shape of the sitting surface 118a is at least partly adjusted to the thigh 208a and/or to the buttock 210a of the person 200a. The sitting surface 118a is curved. The sitting surface 118a is concavely curved and/or bent.

It is also conceivable that a wearable sitting posture assisting device comprises only one seat unit, in particular a common seat unit of two leg units. In particular, in this case it is conceivable that the seat unit is saddle-shaped and/or implemented in the manner of a saddle, in particular arranged between the legs of a person.

The wearable sitting posture assisting device 100a comprises a lower leg 124a. The lower leg 124a comprises a lower leg support 126a. The lower leg 124a has a lower leg longitudinal axis 128a. The lower leg longitudinal axis 128a is oriented perpendicularly to the sitting direction 134a. The lower leg longitudinal axis 128a and the upper leg longitudinal axis 110a are arranged in a common plane. The lower leg support 126a has a main extension direction which is oriented parallel to the lower leg longitudinal axis 128a. The lower leg longitudinal axis 128a is oriented parallel to a main extension direction of a shank 212a of the leg 204a of the person 200a, in particular when the person 200a sits, and/or partly sits and/or sits and/or stands while wearing the wearable sitting posture assisting device 100a.

The upper leg 106a and the lower leg 124a define a sitting angle 130a. The sitting angle 130a is an angle included by the upper leg longitudinal axis 110a and the lower leg longitudinal axis 128a. The sitting angle 130a is similar or identical to an angle between the thigh 208a and the shank 212a of the person 200a. The sitting angle 130a having a value between 60° and 130°, in particular a value of approximately 90°, corresponds to different sitting postures or to at least one sitting posture. The sitting angle 130a having a value between 130° and 170° corresponds to different partly sitting postures. In case the person 200a is standing while wearing the wearable sitting posture assisting device 100a, the sitting angle 130a has a value between 160° and 180°, in particular a value of approximately 180°. In case the person 200a is walking while wearing the wearable sitting posture assisting device 100a, the sitting angle 130a may significantly differ from 180°, in particular in case the person 200a bends his knee 202a. In the sitting posture and/or in the partly sitting posture and/or when standing the sitting angle 130a and an analogously defined additional sitting angle of the additional leg unit 104a are advantageously identical. However, it is also conceivable that the person 200a may sit on the wearable sitting posture assisting device 100a in a sitting posture or a partly sitting posture with the sitting angle 130a and the additional sitting angle differing, in particular by up to 5°, by up to 10°, by up to 15°, by up to 20°, by up to 30°, by up to 40°, or more. When the person 200a is walking while wearing the wearable sitting posture assisting device 100a, the sitting angle 130a and the additional sitting angle may significantly differ, for instance in case the person 200a bends one of his knees 202a more than his other knee 202a.

The leg unit 102a comprises a knee joint 131 a, which pivotably connects the upper leg 106a to the lower leg 124a. The knee joint 131 a connects the upper leg 106a to the lower leg 124a pivotably about a knee joint axis 132a. The knee joint axis 132a is oriented perpendicularly with respect to the upper leg longitudinal axis 110a. The knee joint axis 132a is oriented perpendicularly to the lower leg longitudinal axis 128a. The knee joint axis 132a is oriented perpendicularly to the sitting direction 134a. The knee joint 131 a is partly implemented integrally with the upper leg support 108a. The knee joint 131 a is partly implemented integrally with the lower leg support 126a. The knee joint 131 a comprises at least one bearing 136a, which connects the upper leg support 108a to the lower leg support 126a.

The leg unit 102a comprises a locking unit 138a which is configured for locking the knee joint 131 a. The locking unit 138a is configured for limiting the sitting angle 130a to a minimum value. The locking unit 138a is configured for allowing the person 200a to choose the minimum value of the sitting angle 130a. In case the locking unit 138a is in a locked state, the person 200a can sit down on the wearable sitting posture assisting device 100a with the sitting angle 130a at its minimum value. The locking unit 138a is configured for being actuated by the person 200a. The locking unit 138a comprises a blocking element 140a. The blocking element 140a is a spring, in particular a gas spring. The blocking element 140a is configured for being lockable at different lengths. The blocking element 140a is connected to the upper leg support 108a. The blocking element 140a is connected to the lower leg support 126a. The blocking element 140a is configured for damping a movement of the upper leg 106a with respect to the lower leg 124a, in particular when the person 200a is sitting down.

The leg unit 102a comprises a ground contact unit 152a. The ground contact unit 152a is connected to the lower leg support 126a. The ground contact unit 152a comprises a ground contact element 154a. When the person 200a sits or partly sits on the wearable sitting posture assisting device 100a, the ground contact unit 152a, in particular the ground contact element 154a, is in contact with the ground. The ground contact unit 152a, in particular the ground contact element 154a, is configured for transmitting a portion of a weight force of the person 200a to the ground. The ground contact element 154a is made of rubber. However, other shapes and/or materials are conceivable for a ground contact element as mentioned above.

When the person 200a sits or partly sits on the wearable sitting posture assisting device 100a, the weight force of the person 200a is at least partly, in particular directly or indirectly, transmitted from the seat unit 112a to the upper leg support 108a; from the upper leg support 108a to the knee joint 131 a; from the knee joint 131 a to the lower leg support 126a; from the lower leg support 126a to the ground contact element 154a; and from the ground contact element 154a to the ground.

In particular, the weight force of the person 200a is additionally transmitted to the ground via a foot and/or shoe 214a of the person 200a. Preferably, the ground contact element 154a is arranged on a rear side of the foot and/or shoe 214a of the person 200a. When the person 200a sits or partly sits on the wearable sitting posture assisting device 100a, the foot and/or shoe 214a of the person 200a is in contact with the ground in addition to the ground contact element 154a. Preferably, the ground contact element 154a is arranged contactlessly with respect to the ground when the person 200a walks and/or stands while wearing the wearable sitting posture assisting device 100a.

The wearable sitting posture assisting device 100a comprises an upper body wearing unit 156a. The person 200a wears the upper body wearing unit 156a on his upper body 216a, which upper body 216a may include hips and/or a waist of the person 200a. The upper body wearing unit 156a comprises a belt 158a. Furthermore, the upper body wearing unit 156a comprises suspenders 160a, 162a. The leg unit 102a is connected to the upper body wearing unit 156a. The additional leg unit 104a is connected to the upper body wearing unit 156a. It is conceivable that an upper body wearing unit comprises no belt and only suspenders, or vice versa. It is also conceivable that a wearable sitting posture assisting device is only connected to legs and/or feet and/or shoes of a person who it is worn by.

The wearable sitting posture assisting device 100a comprises a body connection unit 10a. The body connection unit 10a is implemented as a thigh connection unit. The body connection unit 10a is configured for connecting to the thigh 208a of the person 200a. In the case shown, the body connection unit 10a is connected to the leg unit 102a, in particular to the seat unit 112a. Alternatively or additionally the body connection unit 10a may be connected to the upper leg 106a, in particular to the upper leg support 108a. In particular, at least a portion of the body connection unit 10a is permanently fixated to the leg unit 102a.

The wearable sitting posture assisting device 100a comprises an additional body connection unit 24a. The additional body connection unit 24a is implemented as a shank connection unit. The additional body connection unit 24a is configured for connecting to the shank 212a of the person. The additional body connection unit 24a is connected to the leg unit 102a. In the case shown, the additional body connection unit 24a is connected to the lower leg 124a, in particular to the lower leg support 126a. At least a portion of the additional body connection unit 24a is permanently fixated to the leg unit 102a.

The body connection unit 10a and the additional body connection unit 24a are configured for connecting to different kinds of body parts. It is conceivable that a body connection unit and an additional body connection unit are configured for connecting to a thigh and a foot of the person.

In addition or alternatively to the body connection unit 10a and/or the additional body connection unit 24a, it is conceivable that the upper body wearing unit 156a is implemented as a body connection unit according to the invention.

In the case shown, the wearable sitting posture assisting device 100a comprises a second body connection unit 12a. The second body connection unit 12a is implemented analogously to the body connection unit 10a. The second body connection unit 12a is connected to the additional leg unit 104a. Furthermore, in the case shown, the wearable sitting posture assisting device 100a comprises a second additional body connection unit 31 a. The second additional body connection unit 31 a is implemented analogously to the additional body connection unit 24a. The second additional body connection unit 31 a is connected to the additional leg unit 104a.

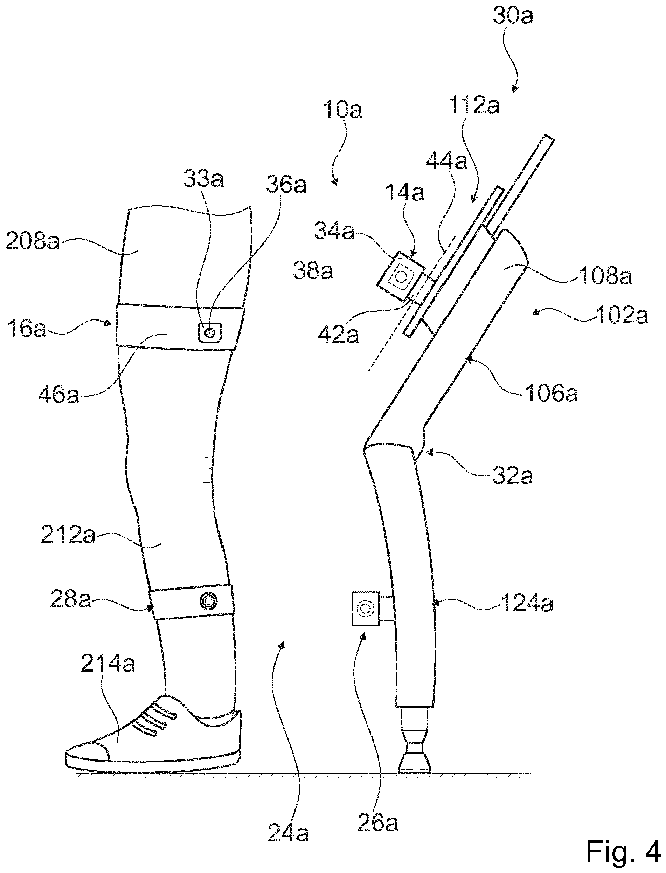

In figures 1 to 3 the body connection units 10a, 12a, 24a, 31 a are shown in a connected state. Figure 4 shows the wearable sitting posture assisting device 100a in a disconnected state from the leg 204a of the person 200a, in a schematic lateral view. In particular, figure 4 shows a disconnected state of the body connection unit 10a and the additional body connection unit 24a. The body connection unit 10a comprises a support element 14a and a body part connector 16a for connecting to a body part that differs from a foot 214a of the person 200a. The body part connector 16a is connectable to and disconnectable from the support element 14a in at least one body part connector wearing state. In the case shown the body part connector 16a is implemented as a thigh connector. Furthermore, the body part connector 16a is connectable to and disconnectable from the support element 14a without tools.

The body part connector 16a comprises a strap element 46a for connecting to a body part of the person 200a, in particular to the thigh 208a. The strap element 46a encompasses the thigh 208a of the person 200a. The strap element 46a may be implemented at least partly flexible and/or deformable and/or elastic and/or permeable to air. Furthermore, the strap element 46a preferably comprises at least one fastener, in particular a quick fastener.

The additional body connection unit 24a comprises an additional support element 26a and an additional body part connector 28a for connecting to a further body part of the person 200a. In the case shown, the additional body part connector 28a is configured for connecting to the shank 212a of the person 200a. The additional body part connector 28a is connectable to and disconnectable from the additional support element 26a. Except for being configured for connecting to a different kind of body part than the body connection unit 10a, the additional body connection unit 24a is implemented analogously to the body connection unit 10a. Therefore, in the following, only the body connection unit 10a is described in more detail. Features described for the body connection unit 10a are to be understood as transferable to the case of the additional body connection unit 24a.

The support element 14a is fixated to the leg unit 102a, in particular to the seat unit 112a. It is also conceivable that the support element 14a is fixated to the upper leg support 108a. It is further conceivable that the support element 14a is at least partly implemented integrally with the seat unit 112a and/or with the upper leg support 108a.

In the normal wearing state, the body part connector 16a is connected to the support element 14a. In particular, a connection between the body part connector 16a and the support element 14a is in the connected state configured for withstanding a force exerted onto the body part connector 16a, in particular a pull force, which is in particular parallel to the sitting direction 134a, of at least 50 N, preferably of at least 100 N.

In figure 4 the body part connector 16a is shown in a body part connector wearing state. In the body part connector wearing state the person 200a wears the body part connector 16a on his thigh 208a. In a method for putting on and/or taking off the wearable sitting posture assisting device 100a, the body part connector 16a is connected to and/or disconnected from the support element 14a in the body part connector wearing state. In particular, the body part connector 16a is connected to the thigh 208a of the person 200a prior to connecting the body part connector 16a to the support element 14a. Furthermore, in particular, the body part connector 16a is disconnected from the support element 14a prior to disconnecting the body part connector 16a from the thigh 208a.

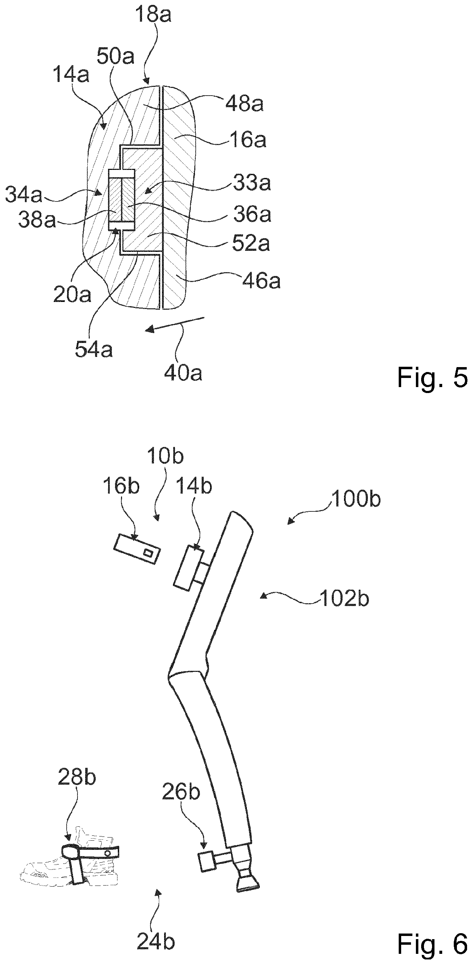

Figure 5 shows a portion of the body connection unit 10a in a schematic sectional view. The body part connector 16a and the support element 14a together implement at least one quick connector 18a. The body part connector 16a comprises a first quick-connection element 33a. The first quick-connection element 33a is connected to the strap element 46a. The first quick-connection element 33a is directly fixated to the strap element 46a. The support element 14a comprises a second quick-connection element 34a. The quick-connection elements 33a, 34a together implement the quick connector 18a.

The leg unit 102a and the support element 14a together implement a functional module 30a of the wearable sitting posture assisting device 100a. The leg unit 102a implements a functional unit 32a of the functional module 30a, the functional unit 32a being configured for implementing at least one posture assisting function for the person 200a in the wearing state. The functional module 30a, in particular the support element 14a, is configured for connecting to and/or disconnecting from the body part connector 16a.

The quick connector 18a is self-fastening and/or self-connecting. In the case shown, the quick connector 18a automatically connects in case the body part connector 16a is brought in a proximity, in particular within no more than 5 cm, of the support element 14a, at least unless the body part connector 16a and/or the support element 14a are prevented from approaching each other further due to a holding force. For instance, the person 200a may connect the body part connector 16a to the support element 14a by moving the body part connector 16a towards the support element 14a, at least into a proximity position of the body part connector 16a, in which a distance between the body part connector 16a and the support element 14a is preferably less than 5 cm, advantageously less than 3 cm, and preferably by subsequently allowing the body part connector 16a to connect to the support element 14a by omitting to exert a counterforce onto the body connector element 16a and/or the support element 14a. Furthermore, in the case shown the quick connector 18a is self-centering. The first quick-connection element 33a and the second quick-connection element 34a are configured for automatically assuming a position relative to each other in a proximity state, in particular in the proximity position, which corresponds to a position of the first quick-connection element 33a and the second quick-connection element 34a relative to each other in a connected state of the quick connector 18a.

The body part connector 16a and the support element 14a together implement a magnetic connection 20a. The quick connector 18a implements the magnetic connection 20a.The first quick-connection element 33a comprises at least one first magnet element 36a. The second quick-connection element 34a comprises at least one second magnet element 38a. The first magnet element 36a and/or the second magnet element 38a may be implemented as magnets and/or as magnetizable elements. In addition, it is conceivable that the magnetic connection 20a comprises a plurality of magnet elements, which may be arranged in connection pairs or in other configurations. For instance, one quick-connection element may comprise a plurality of magnets which are configured for connecting to only one specific magnet element of the other quick-connection element. It is further conceivable that the magnetic connection is a connection in addition to a quick connector, wherein, in particular, the quick connector and the magnetic connection are arranged at two different positions and/or separate from each other.

In the case shown the magnet elements 36a, 38a are implemented as magnets. The first magnet element 36a and the second magnet element 38a are contacting each other in the connected state of the body part connector 16a. In the case shown a connection direction 40a of the first magnet element 36a and the second magnet element 38a is oriented inclined or perpendicular to a plane defined by the sitting direction 134a and the upper leg longitudinal axis 110a. It is also conceivable that the connection direction 40a is oriented parallel to this plane. In the proximity state of the body part connector 16a the magnet element 36a and the second magnet element 38a attract each other thus implementing the self-centering.

The support element 14a is implemented at least partly macroscopically deformable. The support element 14a is configured for being deformed when connecting and/or disconnecting the body part connector 16a and/or when the person 200a is moving his leg 204a or foot and/or shoe 214a while wearing the wearable sitting posture assisting device 100a. In the case shown, the support element 14a comprises an attachment element 42a, which connects the second quick-connection element 34a to the leg unit 102a, in particular to the seat unit 112a. The attachment element 42a is at least partly macroscopically deformable. In the case shown the attachment element 42a is made of rubber or flexible plastic. The attachment element 42a movably mounts the second quick-connection element 34a to the leg unit 102a. In the proximity state the second quick-connection element 34a tilts towards the first connection element 33a around a tilt axis 44a defined by the attachment element 42a.

The body part connector 16a and the support element 14a together implement at least one form-fit connection 22a. In the case shown, the quick connector 18a implements the form-fit connection 22a. The quick connector 18a is configured for providing at least one inadvertent-error-prevention constraint. The quick connector 18a implements a poka yoke connection. The quick-connection elements 33a, 34a implement the form-fit connection 22a, which is in particular of a poka yoke type. In particular, the body part connector 16a is not compatible with the additional body connection unit 24a and/or the additional body part connector 28a is not compatible with the body connection unit 10a. In the case shown the second quick-connection element 34a comprises a recess element 48a which defines a recess 50a for the first quick-connection element 33a. In the connected state the first quick-connection element 33a is at least partly arranged inside the recess 50a. The first quick-connection element 33a comprises a protrusion element 52a which is configured for insertion into the recess 50a. In particular, a cross section of the protrusion element 52a corresponds to a cross section of the recess 50a. The corresponding cross sections of the protrusion element 52a and the recess 50a implement the inadvertent-error-prevention constraint and/or the form-fit connection 22a. The first magnet element 36a is connected to, in particular directly fixated to, the protrusion element 52a. The second magnet element 38a is connected to, in particular directly fixated to, the recess element 48a. The second magnet element 38a delimits the recess 50a. The protrusion element 52a and the recess element 48a implement at least one form-fit connection surface 54a, which is oriented perpendicularly with respect to the connection direction 40a. The form-fit connection surface 54a corresponds to an outer surface, in particular a shell surface, of the protrusion element 52a. The form-fit connection surface 54a is configured for transmitting a pull and/or push force exerted onto the body part connector 16a to the support element 14a.

Figure 6 shows a further exemplary embodiment of the invention. The following description is substantially limited to the differences between the exemplary embodiments, wherein regarding structural elements, features and functions that remain the same the description of the other exemplary embodiment of figs. 1 to 5 may be referred to. For distinguishing the exemplary embodiments, the letter a of the reference numerals in the exemplary embodiment of figs. 1 to 5 has been substituted by the letter b in the reference numerals of the exemplary embodiment of figure 6. Regarding structural elements having the same denomination, in particular regarding structural elements having the same reference numerals, principally the drawing and/or the description of the other exemplary embodiments, in particular of the exemplary embodiment of figs. 1 to 5, may be referred to.

Figure 6 shows an alternative wearable sitting posture assisting device 100b, in a schematic lateral view. The alternative wearable sitting posture assisting device 100b comprises a leg unit 102b. The alternative wearable sitting posture assisting device 100b comprises a body connection unit 10b and an additional body connection unit 24b. The body connection unit 10b comprises a support element 14b and a body part connector 16b for connecting to at least one body part which differs from a foot of a person. The body part connector 16b is connectable to and/or disconnectable from the support element 14b. The support element 14b is connected to the leg unit 102b. The additional body connection unit 24b comprises an additional support element 26b and an additional body part connector 28b. The additional body part connector 28b is connectable to and/or disconnectable from the additional support element 26b. The additional support element 26b is connected to the leg unit 102b.

The body connection unit 10b is implemented as a thigh connection unit. The body part connector 16b is implemented as a thigh connector. The additional body connection unit 24b is implemented as a foot connection unit. The additional body part connector 28b is implemented as a foot connector.

## Claims

1. A wearable sitting posture assisting device, comprising at least one body connection unit (10a, 12a; 10b), the body connection unit (10a, 12a; 10b) featuring at least one support element (14a; 14b) and at least one body part connector (16a; 16b) for connecting to at least one body part which differs from a foot of a person (200a), **characterized in that** the body part connector (16a; 16b) is connectable to and/or disconnectable from the support element (14a; 14b) in at least one body part connector wearing state.

2. The wearable sitting posture assisting device according to claim 1, **characterized in that** the body part connector (16a) and the support element (14a) together implement at least one quick connector (18a).

3. The wearable sitting posture assisting device according to claim 2, **characterized in that** the quick connector (18a) is self-fastening and/or self-connecting.

4. The wearable sitting posture assisting device according to claim 2 or 3, **characterized in that** the quick connector (18a) is configured for providing at least one inadvertent-error-prevention constraint.

5. The wearable sitting posture assisting device according to any one of the preceding claims, **characterized in that** the body part connector (16a) and the support element (14a) together implement at least one magnetic connection (20a).

6. The wearable sitting posture assisting device according to any one of the preceding claims, **characterized in that** the body part connector (16a) and the support element (14a) together implement at least one form-fit connection (22a).

7. The wearable sitting posture assisting device according to any one of the preceding claims, **characterized in that** the body part connector (16a) is implemented as a thigh connector and/or as a shank connector.

8. The wearable sitting posture assisting device according to any one of the preceding claims, **characterized in that** the support element (14a) is at least partly macroscopically deformable.

9. The wearable sitting posture assisting device according to any one of the preceding claims, **characterized by** at least one leg unit (102a; 102b), to which the support element (14a; 14b) is connected.

10. The wearable sitting posture assisting device according to claim 9, **characterized by** at least one additional leg unit (104a), which is implemented at least substantially identically to the leg unit (102a).

11. The wearable sitting posture assisting device according to any one of the preceding claims, **characterized by** at least one additional body connection unit (24a; 24b) featuring at least one additional support element (26a; 26b) and at least one additional body part connector (28a; 28b) for connecting to at least one additional body part of the person (200a), wherein the additional body part connector (28a; 28b) is connectable to and/or disconnectable from the additional support element (26a; 26b).

12. The wearable sitting posture assisting device according to claim 11, **characterized in that** the body connection unit (10a; 10b) and the additional body connection unit (24a; 24b) are configured for connecting to different kinds of body parts.

13. A body part connector (16a; 16b) for a wearable sitting posture assisting device (100a) according to any one of the preceding claims.

14. A functional module (30a), in particular configured for a wearable sitting posture assisting device (100a) according to any one of claims 1 to 12, comprising at least one functional unit (32a), the functional unit (32a) being configured for implementing at least one posture assisting function for a person (200a) in at least one wearing state, and comprising at least one support element (14a) which is configured for connecting to and/or disconnecting from at least one body part connector (16a) in at least one body part connector wearing state.

15. A method for putting on and/or taking off a wearable sitting posture assisting device (100a), in particular according to any one of claims 1 to 12, comprising at least one support element (14a) and at least one body part connector (16a), wherein the body part connector (16a) is connected to and/or disconnected from the support element (14a) in at least one body part connector wearing state.
